# EUROPÄISCHE PATENTANMELDUNG

(11) **EP 1 243 327 A1**
(43) Veröffentlichungstag der Anmeldung: **25.09.2002**
(21) Anmeldenummer: 01106789.9
(22) Anmeldetag: 19.03.2001
(51) Int. Cl.: B01J 19/00, G01N 33/50

(54) **Oberfläche mit einem Muster aus Zellen und Verfahren zu deren Herstellung**

(71) Anmelder: BioChip Technologies GmbH, 79108 Freiburg i. Br. (DE)
(72) Erfinder: Laufen, Thomas, 79100 Freiburg (DE); Maier, Rainer, 79100 Freiburg (DE)
(74) Vertreter: Stürken, Joachim

(57) **Zusammenfassung**

Die Erfindung betrifft eine Oberfläche mit einem Muster aus kovalent darauf in Monolayern immobilisierten Zellen, Zellfragmenten, membranösen Zellorganellen oder Membranvesikeln sowie ein Verfahren zu deren Herstellung.

## Beschreibung

Die Erfindung betrifft eine Oberfläche mit einem Muster aus kovalent darauf in Monolayern immobilisierten Zellen, Zellfragmenten, membranösen Zellorganellen oder Membranvesikeln, sowie ein Verfahren zu deren Herstellung.

Eine Aufstellung der in der folgenden Schilderung des Standes der Technik in Bezug genommenen Literaturstellen mit genaueren bibliographischen Angaben findet sich am Ende dieser Beschreibung.

In den letzten Jahren haben in der Analytik Mikrotechniken immer mehr an Bedeutung gewonnen und es sind zahlreiche Festphasensysteme auf der Grundlage von sich selbst organisierenden Monoschichten (engl. »*self-assembled monolayers*«, »SAMs«) aus bifunktionellen Molekülen (engl. »*Crosslinker*« bzw. *»Linker*«) entwickelt worden, über die spezifisch Probenmoleküle an die Oberfläche des festen Trägers gekoppelt bzw. konjugiert werden, an der dann auch der Nachweis mit Hilfe von geeigneten Markierungen (beispielsweise radioaktiv, gefärbt, fluoreszierend) erfolgt.

Für diese Systeme hat sich in Analogie zu den elektronischen Mikrochips die Bezeichnung Sensorchips eingebürgert. Im Falle der Konjugation von biologischen Molekülen (sog. »Biokonjugation«) an solche Sensorchips, beispielsweise Oligonukleotiden oder Proteinen wie Antikörpern, spricht man auch von Biochips. Die Kopplung an die Trägeroberfläche kann direkt oder indirekt erfolgen. Ein Beispiel für eine indirekte Kopplung ist die Kopplung einer nachzuweisenden Nukleinsäuresequenz durch Hybridisierung an ein immobilisiertes, komplementäres Oligonukleotid als Sonde. In diesem Fall hat die Verwendung der Sonde noch den Vorteil der natürlichen Spezifität der Wechselwirkung biologischer Makromoleküle.

Typischerweise werden zur Herstellung von Sensorchips Oberflächen aus Metall- bzw. Halbmetalloxiden, wie z.B. Aluminiumoxid, Quarzglas, Glas, in eine Lösung von mono- oder bifunktionellen Molekülen bzw. Vernetzern (sog. »Crosslinker« bzw. »Linker«), die beispielsweise eine Halogensilan- (z.B. Chlorsilan-) oder Alkoxysilangruppe zur Kopplung an die Trägeroberfläche aufweisen, getaucht, so daß sich eine sich selbst organisierende Monoschicht (SAM) bildet. Diese weist in diesem Fall eine Dicke von wenigen Ångström auf. Die Kopplung der Linker an die Proben- oder Sondenmoleküle erfolgt über eine geeignete weitere funktionelle Gruppe, beispielsweise eine Amino- oder Epoxygruppe. Geeignete mono- oder bifunktionelle Linker für die Kopplung einer Vielzahl von Proben- oder Sonden-Molekülen, insbesondere auch biologischen Ursprungs, an eine Vielzahl von Trägeroberflächen sind dem Fachmann gut bekannt, vgl. beispielsweise »Bioconjugate Techniques« von G. T. Hermanson, Academic Press 1996.

Diese Technik läßt sich auch auf Zellen übertragen, und es sind derzeit verschiedene Verfahren bekannt, die es ermöglichen Zellen an Oberflächen zu koppeln und beispielsweise immunologisch nachzuweisen.

Von der Firma BIACore, Freiburg, Deutschland ist ein Sensorchip (CM5) erhältlich, der auf seiner Oberfläche Dextranfibrillen trägt, die Carboxymethylgruppen tragen. Durch EDC/-NHS-Crosslinking (EDC = 1-Ethyl-3-(3dimethylaminopropyl)carbodiimid, NHS = N-Hydroxysuccinimidester) können z.B. IgG (1) und Fibronektin (2) an den Chip gekoppelt werden, über die dann Bakterienzellen auf dem Chip immobilisiert werden können. Bei den genannten, zur Kopplung verwendeten Chemikalien handelt es sich um ein Carbodiimid und allgemein ein Nukleophil, beispielsweise ein Nukleophil, welches das Carbodiimid. substituiert und die Carboxygruppe in einen aktivierten Ester überführt, z.B. ein cyclisches Imid wie NHS, S-NHS (= Sulfo-NHS) und HOAt (1-Hydroxyazabenzotriazol). Beide Stoffklassen sind sehr verbreitet zur Kopplung von Carboxy- und Aminogruppen aneinander, d.h. z.B. einer Carboxylmethylgruppe der Dextranfibrillen auf dem BIACore-Sensorchip, an eine Aminogruppe eines Proteins.

Außerdem ist es möglich durch Kopplung von Concanavalin A (1) Erythrozyten auf dem Chip zu immobilisieren. Die so immobilisierten Zellen können dann auf Bindung an Biomoleküle untersucht werden, wobei der observierte Parameter die Änderung des Plasmonresonanzspektrums ist.

Bei einem weiteren Verfahren, ebenfalls von der Firma BIACore, könnten Zellen durch hydrophobe Wechselwirkung mit der Chipoberfläche interagieren. Der HPA-Chip trägt einen sog. »self assembling monolayer« (SAM) an den Liposomen gebunden werden können. Dieses Verfahren wäre auch zur Kopplung von Zellen anwendbar.

Außerdem ist die Kopplung von Zellen an Poly-L-Lysin-Oberflächen sehr weit verbreitet, z.B. auf »Poly-Prep-Slides«, Sigma, P-0425. Die Zellen werden jedoch nicht kovalent an diese Oberfläche gebunden und haften nach eigenen Versuchen schlechter als Zellen, die erfindungsgemäß gekoppelt wurden.

Mit den beschriebenen Verfahren des Standes der Technik ist es aber nicht möglich, verschiedene Zelltypen unter Musterbildung, d.h. in Form eines adressierbaren »Arrays«, auf einfache und schnelle Weise direkt als Monolayer auf eine Oberfläche, z.B. einen Chip, aufzubringen und kovalent zu immobilisieren. Mit einer solchen Oberfläche bzw. einem solchen Chip ließe sich eine Probe mit verschiedenen Zelltypen in einem Ansatz zu untersuchen. Bei den Chips nach dem Stand der Technik haften die Zellen über Wechselwirkungen, die schwächer sind als eine kovalente Bindung.

Aufgabe der Erfindung ist die Bereitstellung einer derartigen Oberfläche bzw. eines derartigen Chips.

Die Erfindung beruht auf der überraschenden Erkenntnis, daß eine derartige Oberfläche schnell und einfach erhältlich ist, indem die Oberflächen oder die Zellen (einschließlich Zellfragmente, membranöse Zellorganellen oder Membranvesikel) mit mindestens einem Vernetzungsmittel (Crosslinker) (im Falle von mehreren Vernetzungsmitteln können diese gleich oder unterschiedlich, mono- oder bifunktional und für den Fall eines bifunktionalen Vernetzers homo- oder heterofuntkional sein) aktiviert und anschließend die Zellen (einschließlich Zellfragmente, membranöse Zellorganellen oder Membranvesikel) unter Musterbildung in Form von Monolayern auf die Oberfläche gedruckt und kovalent an dieser immobilisiert werden.

Im folgenden ist der Einfachheit halber auch nur von Zellen die Rede, es sind aber stets auch Zellfragmente, membranöse Zellorganellen oder Membranvesikeln gemeint.

Vorteilhaft ist dabei, daß nicht nur auf einfache Weise, nämlich durch Drucken, eine Aufbringung und kovalente Immobilisierung von Zellen in Form von Monolayern auf Oberflächen unter Musterbildung möglich ist, sondern das diese außerdem nicht in einer den spezifischen Nachweis von Oberflächenmolekülen, z.B. Proteinen, behindernden oder sogar unmöglich machenden Weise verändert werden. Beispielsweise bleiben zelluläre Membranproteine aufgrund der milden Immobilisierungsbedingungen unverändert, so daß diese bzw. die Zellen immunologisch nachweisbar bleiben.

Dieses Ergebnis ist überraschend, da eigentlich zu erwarten war, daß beim Drucken die Zellen beispielsweise nicht an die Nadel eines Nadeldruckers (Pinprinters) binden oder sie verkleben, oder die Düsen eines »Nonimpact«-Druckers/kontaktlosen Druckers verstopfen und/oder sich keine Monolayer auf der Oberfläche bilden.

Es wichtig, das die gekoppelten Zellen als Monolayer auf der Oberfläche, beispielsweise des Chips, haften, weil sie so bei weiterer Verarbeitung nicht durch Scherkräfte von der Oberfläche, beispielsweise des Chips, gespült werden, die Teile der Zelloberfläche, über die ein Nachweis erfolgen soll, nicht durch Bildung eines Aggregats blockiert werden und die Anzahl der nachzuweisenden Teile der Oberfläche direkt proportional zur bedeckten Oberfläche, z.B. des Biochips, ist. Nur wenn alle drei Voraussetzungen erfüllt sind, ist es möglich, an den gekoppelten Zellen quantitative Messungen vorzunehmen.

Außerdem ist überraschend, daß die Zellen als Ganzes, d.h. in intakter Form, kovalent gebunden an der Oberfläche, beispielsweise des Chips, haften. Es hätte auch geschehen können, daß die Teile der Zelloberfläche, über die die Zellen an die Oberfläche binden, beispielsweise die Oberfläche des Chips, herausgerissen werden.

Die Erfindung betrifft ferner ein Verfahren zum Nachweis von Membranbestandteilen wie Oberflächenproteinen, Zuckern, Sterolen und Lipiden auf Zellen (oder Zellfragmenten, membranösen Zellorganellen oder Membranvesikeln), bei dem ein(e) erfindungsgemäße(r) Oberfläche/Chip mit einem oder mehreren, mit den jeweils nachzuweisenden Membranbestandteilen, z.B. Oberflächenproteinen, spezifisch reagierenden Nachweisreagenzien in Kontakt gebracht wird, ein medizinisches oder diagnostisches Gerät, das eine(n) erfindungsgemäße(n) Oberfläche/Chip aufweist sowie einen Kit zur Verwendung mit einem solchen Gerät, der eine(n) erfindungsgemäße(n) Oberfläche/Chip und entsprechende Nachweisreagenzien enthält.

Neben qualitativen oder quantitativen Messungen für beispielsweise diagnostische Zwecke gestatten die erfindungsgemäßen Oberflächen oder Chips auch strukturelle und funktionelle Untersuchungen an Membranen, da auf einfache Weise durch Wahl geeigneter Crosslinker eine Orientierung der Membran auf der Oberfläche möglich ist, so daß beispielsweise die Verteilung von Oberflächenmolekülen auf der Außenseite oder Innenseite (d.h. zum Zellinneren hin) der Membran bestimmt werden kann oder Untersuchungen an Transmembranproteinen oder Rezeptoren und deren Wechselwirkungen mit Liganden möglich sind.

Es ist ferner klar, daß mit den erfindungsgemäßen Oberflächen/Chips nicht nur die Membranmoleküle der daran immobilisierten Zellen untersucht werden können, sondern auch deren Inhalt, beispielsweise das Cytosol, der Kern oder Zellorganellen bzw. deren Inhalt.

Weitere vorteilhafte und/oder bevorzugte Ausführungsformen der Erfindung sind Gegenstand der Unteransprüche.

Die erfindungsgemäß geeigneten Oberflächen unterliegen keinen besonderen Beschränkungen hinsichtlich der chemischen Beschaffenheit, der Form oder der Abmessungen, sofern sie zum Bedrucken geeignet sind.

Beispielsweise können an der Oberfläche freie Epoxy-, Carboxy-, Amino- Aldehyd-, Cyano, Isothiocyano-, Thiocyano- oder Thiolgruppen vorhanden sein, an die ein bifunktioneller Vernetzer zur Aktivierung (und zur späteren kovalenten Immobilisierung der Zellen) kovalent gebunden werden kann oder an die mit einem bifunktionellen Vernetzer entsprechend aktivierte Zellen kovalent immobilisiert werden können. Diese Gruppen können auf der Oberfläche schon von vornherein vorhanden sein wie zum Beispiel auf verschiedenen Membranen oder Polymeren oder durch eine entsprechende Beschichtung aufgebracht werden wie zum Beispiel durch Silanisierung, Bedampfung oder Pfropfung bzw. »Grafting« von Polymer- oder Glaschips, Membranen oder Metalloberflächen. Es können natürlich nicht nur feste Oberflächen verwendet werden, sondern auch »halbfeste«, beispielsweise gelartige Oberflächen, sofern darauf das gedruckte Zellmuster nicht verläuft oder darin absorbiert wird.

Die Vernetzer (Crosslinker) unterliegen ebenfalls keinen besonderen Beschränkungen, und dem Fachmann sind geeignete Vernetzer (Crosslinker) zur Kopplung einer Vielzahl von Biomolekülen an eine Vielzahl von Trägeroberflächen gut bekannt, vgl. beispielsweise »Bioconjugate Techniques« von G. T. Hermanson, Academic Press 1996. Zur Kopplung können ein oder mehrere homo- oder bifunktionale Vernetzer und für den Fall eines binfunktionalen Vernetzers Homo- oder Heterofunktionale verwendet werden.

Im Falle von Oberflächen mit freien Epoxy-, Carboxy- oder Aminogruppen eignen sich als Vernetzer (Crosslinker) beispielsweise ein Carbodiimid und allgemein ein Nukleophil, beispielsweise ein Nukleophil, welches das Carbodiimid substituiert und die Carboxygruppe in einen aktivierten Ester überführt, z.B. ein cyclisches Imid wie NHS (= N-Hydroxysuccinimidester), S-NHS (= Sulfo-NHS) und HOAt (= 1-Hydroxyazabenzotriazol).

Als Vernetzer (Crosslinker) eignen sich hinsichtlich des Carbodiimids beispielsweise EDC (= 1-Ethyl-3-(3dimethylaminopropyl)carbodiimid oder DICD (= Diisopropylcarbodiimid) und hinsichtlich des Nucleophils beispielsweise NHS, S-NHS oder HOAt.

Das Carbodiimid, beispielsweise EDC oder DICD, und das Nukleophil, beispielsweise NHS oder S-NHS, können grundsätzlich unabhängig voneinander verwendet werden, d.h. es ist keine Kombination erforderlich. Die Kombination von beispielsweise EDS/S-NHS wird aber hier bevorzugt, weil sich eine wesentlich bessere Immobilisierung als bei der Verwendung nur einer der beiden Komponenten ergibt und S-NHS (gegenüber NHS) den Vorteil hat, das es wasserlöslich ist und sich sonst keine weitere Eigenschaft ändert.

Es ist ferner klar, daß es nicht darauf ankommt, ob vor dem Drucken die Oberfläche oder die Zellen mit den Vernetzer (Crosslinkern) aktiviert wird/werden. Im Falle der Aktivierung der Oberfläche kann sich der Aktivierung ein Waschschritt zur Entfernung von überschüssigem Vernetzungsmittel anschließen. Falls die Zellen aktiviert werden, wird die Konzentration des Vernetzungsmittels zweckmäßigerweise so gewählt, daß die Zellen nicht miteinander vernetzen. Geeignete Konzentrationsbereiche lassen sich mit einfachen Versuchen bestimmen.

Zum Drucken kann beispielsweise ein Nadeldrucker oder ein »Nonimpact«-Drucker/kontaktloser Drucker verwendet werden. Da Nadeldrucker weitverbreitet und besonders unanfällig gegen Störungen sind (es können keine Düsen verstopfen) werden diese hier bevorzugt verwendet. Zum Drucken von Mustern (»patterned arrays«) eignet sich z.B. eine Nadel mit einem Spot-Durchmesser 200 µm. Außerdem können Nadeln verwendet werden, die Spots mit anderen Durchmessern drucken. Eine untere Grenze liegt derzeit bei ca. 100 µm, eine obere Grenze wurde auch mit 600 um nicht erreicht.

Hinsichtlich der immobilisierbaren Zellen bestehen ebenfalls keine besonderen Beschränkungen, es können beliebige Zellen prokaryotischen oder eukaryotischen Ursprungs verwendet werden. Ebenfalls verwendet werden können Zellfragmente oder Zelltrümmer, Membranvesikel (z.B. »Inverted Vesicels«, ER-Vesikel, membranöse Organellen (Mitochondrien, Plastiden, Vakuolen etc.) oder Fragmente oder Trümmer davon.

Die Nachweisreagenzien unterliegen keinen besonderen Beschränkungen und können einen oder mehrere unmarkierte oder markierte polyklonale oder monoklonale Antikörper, chimäre Antikörper oder »Single-chain«-Antikörper oder funktionelle Fragmente oder Derivate davon umfassen. Außerdem können im Falle von spezifisch Liganden bindenden Oberflächenstrukturen (beispielsweise Rezeptoren) diese Liganden (ggf. nach entsprechender Markierung) verwendet werden, z.B. entsprechende Hormone für Hormonrezeptoren, entsprechende Biomoleküle für ABC-Transporter, selektive Poren oder Kanäle.

Die Markierung kann beliebig und beispielsweise ausgewählt sein unter radioaktiven, farbigen, fluoreszierenden, biolumineszierenden, chemilumineszierenden oder phosphoreszierenden Markierungen oder auf einem Enzym, einem Antikörper oder einem funktionellen Fragment oder Derivat davon oder einem auf einem Protein-A/Gold-, Protein-G/Gold oder Avidin/Streptavidin/Biotin-System beruhen.

Es ist klar, daß die eigentliche Nachweisreaktion direkt oder indirekt erfolgen kann. Im Falle von beispielsweise Antikörpern als Nachweisreagenz könnte der direkte Nachweis auf der Bindung von markierten spezifischen Antikörpern beruhen. Ein indirekter Nachweis könnte auf der Bindung eines unmarkierten primären Antikörpers an ein Antigen (z.B. ein Oberflächenprotein) und der sich anschließenden Bindung eines markierten sekundären Antikörpers gegen den primären Antikörper (sog. »Anti-Antikörper«) beruhen (sog. »Sandwich«-Verfahren).

Im folgenden wird die Erfindung ohne Beschränkung detaillierter beschrieben.

Die Immobilisierung beliebiger Zellen, Zellfragmente, membranöser Zellorganellen oder Membranvesikel kann beispielsweise folgendermaßen erfolgen.

Zum Fixieren der Zellen werden ca. 8•10⁶ Zellen in 1 ml 150 mM PBS, pH 7.2 aufgenommen. Unter langsamen Vortexen wird 10% Formaldehyd, 10% Paraformaldehyd oder 10% Glutaraldehyd zu einer Endkonzentration von 0.5% (v/v) zugegeben. Die Zellen werden 30 min auf Eis inkubiert, alle 10 min kurz »gevortext« und schließlich bei max. 400g 5 min bei 4°C pelletiert. Anschließend werden die Zellen 2mal in 1 ml 150 mM PBS pH 7.2 gewaschen. Die so behandelten Zellen sind bei 4°C bis zu zwei Wochen lagerfähig. Auf den Fixierungsschritt kann verzichtet werden, die Zellen verlieren dann allerdings nach kurzer Zeit (wenige Tage) ihre Immunogenität.

Zum Drucken werden 8•10⁶ Zellen mit max. 400 x g bei 4°C 5 min pelletiert, so daß ein Pellet mit ca. 50 µl Volumen entsteht. Das Pellet wird in 200 µl 150 mM PBS pH 7.2 aufgenommen, das zusätzlich 6.3 mM EDC und 0.92 mM S-NHS zum Vernetzen (»crosslinking«) enthält und 5 min bei Raumtemperatur inkubiert. Als Vernetzer können, wie oben beschrieben, auch andere Carbodiimide oder Nukleophile verwendet werden. Es können auch andere Verhältnisse von EDC und S-NHS verwendet werden. Es ist auch möglich, auf eine der beiden Komponenten, das Carbodiimid oder das Nukleophil, zu verzichten. Allerdings ergibt sich bei kombinierter Anwendung eine wesentlich bessere Immobilisierung. Nach der Inkubation werden die Zellen erneut pelletiert und der Überstand verworfen. Das so gewonnene Pellet kann direkt zum Drucken verwendet werden.

Zum Drucken wird das Pellet in ELISA Platten überführt. 384-Well-Platten (»*well*« = *engl*. »Vertiefungen«) werden mit 40 µl Pellet pro Well befüllt, 96-Well-ELISA-Platten werden mit 40 µl Pellet pro Well befüllt. Unter Verwendung eines Array-Druckers Modell Omnigrid, GeneMachines, und einer Nadel mit 200 µm Spot-Durchmesser werden die Zellen in einem Array aufgedruckt, wobei eine Nadel nach der Beladung ca. 10 Spots absetzen kann.

Zum Koppeln der Zellen und zum Blocken der Chipoberfläche werden die bedruckten Chips unmittelbar nach dem Druck zunächst zwei Stunden bei Raumtemperatur inkubiert, um die Zellen kovalent an den Chip zu koppeln. Dabei können die Zellen trocken werden oder feucht bleiben. Anschließend werden die Chips 30 min in 100 mM Boratpuffer bei pH 9.5 inkubiert, um verbliebene aktivierte Ester zu hydrolysieren. Die Hydrolyse kann auch mit einem anderen anorganischen Puffer bei diesem pH durchgeführt werden.

Danach werden die Zellen 2mal 5 min in 150 mM PBS pH 7.2 gewaschen. Zum Blocken der Oberfläche werden die Chips dann 1 h in 3% BSA in 150 mM PBS bei pH 7.2 inkubiert und danach 2mal 5 min in Cova-Puffer (2 M NaCl, 10 % MgSO₄, 150 mM PBS pH 7.2, 0,1 % Tween 20) gewaschen.

Die Zelloberflächenproteine werden durch einen Erstantikörper (Primärantikörper) nachgewiesen (im hier beschriebenen Fall mit einem monoklonalen α-myc-Antikörper, 0.003 mg/ml, 1 h Inkubationszeit bei Raumtemperatur). Der Chip wird erneut 2mal 5 min in Cova-Puffer gewaschen, worauf der Erstantikörper durch einen markierten Zweitantikörper (Sekundärantikörper oder Anti-Antikörper) nachgewiesen wird. (Im hier beschriebenen Fall Esel-α-Maus-Cy5, 0.005 mg/ml, 1 h Inkubationszeit bei Raumtemperatur). Der Chip wird erneut 2mal 5 min in Cova-Puffer gewaschen, vorsichtig mit Stickstoff trockengeblasen und ausgelesen.

Die besten Ergebnisse wurden mit fixierten (wie oben beschrieben) und mit mittels EDC/S-NHS kovalent auf dem Chip immobilisierten Zellen erzielt.

### Bibliographie

1. BIAtechnology Note 103, BIAcore AB
2. Holmes, S. et al. (1994), präsentiert auf dem 4. European BIAsymposium, Heidelberg

## Patentansprüche

1. Oberfläche mit einem Muster aus kovalent darauf in Monolayern immobilisierten Zellen, Zellfragmenten, membranösen Zellorganellen oder Membranvesikeln.

2. Oberfläche mit einem Muster aus kovalent darauf in Monolayern immobilisierten Zellen, Zellfragmenten, membranösen Zellorganellen oder Membranvesikeln, erhältlich nach einem Verfahren, bei dem
a) eine Oberfläche oder Zellen, Zellfragmente, membranöse Zellorganellen oder Membranvesikeln mit einem Vernetzer (Crosslinker) aktiviert wird/werden, und anschließend
b) die Zellen, Zellfragmente, membranösen Zellorganellen oder Membranvesikeln unter Musterbildung in Form von Monolayern auf die Oberfläche gedruckt und kovalent an dieser immobilisiert werden.

3. Verfahren zur Herstellung einer Oberfläche mit einem Muster aus kovalent darauf in Monolayern immobilisierten Zellen, Zellfragmenten, membranösen Zellorganellen oder Membranvesikeln nach Anspruch 1 oder 2, bei dem
a) eine Oberfläche oder Zellen, Zellfragmente, membranöse Zellorganellen oder Membranvesikeln mit mindestens einem bifunktionellen Vernetzer aktiviert wird/werden, und anschließend
b) die Zellen, Zellfragmente, membranösen Zellorganellen oder Membranvesikeln unter Musterbildung in Form von Monolayern auf die Oberfläche gedruckt und kovalent an dieser immobilisiert werden.

4. Oberfläche, erhalten nach einem Verfahren nach Anspruch 3.

5. Oberfläche oder Verfahren nach einem der vorstehenden Ansprüche, wobei die Oberfläche Amino-, Carboxy- oder Epoxygruppen aufweist und als Vernetzer (Crosslinker) ein Carbodiimid und ein Nukleophil verwendet werden.

6. Oberfläche oder Verfahren nach Anspruch 5, wobei das Carbodiimid 1-Ethyl-3-(3dimethylaminopropyl)carbodiimid oder Diisopropylcarbodiimid und das Nukleophil Sulfo-N-Hydroxysuccinimidester ist.

7. Oberfläche oder Verfahren nach einem der vorstehenden Ansprüche, wobei die Zellen prokaryotischen oder eukaryotischen Ursprungs sind.

8. Oberfläche oder Verfahren nach einem der vorstehenden Ansprüche, wobei zum Drucken ein Nadeldrucker verwendet wird.

9. Oberfläche nach einem der vorstehenden Ansprüche, die Teil eines Sensorchips oder Biochips ist.

10. Verfahren zum Nachweis von Oberflächenmolekülen auf Zellen, Zellfragmenten, membranösen Zellorganellen oder Membranvesikeln oder von Molekülen im Inneren der Zellen, bei dem eine Oberfläche nach einem der vorstehenden Ansprüche mit einem oder mehreren, mit den jeweils nachzuweisenden Oberflächenmolekülen oder Molekülen aus dem Inneren der Zellen spezifisch reagierenden Nachweisreagenz(ien) in Kontakt gebracht wird.

11. Verfahren nach Anspruch 10, wobei die Nachweisreagenzien einen oder mehrere unmarkierte oder markierte polyklonale oder monoklonale Antikörper, chimäre Antikörper oder »Singlechain«-Antikörper oder funktionelle Fragmente oder Derivate davon umfassen.

12. Verfahren nach Anspruch 11, wobei die Markierung ausgewählt ist unter radioaktiven, farbigen, fluoreszierenden, biolumineszierenden, chemilumineszierenden oder phosphoreszierenden Markierungen oder auf einem Enzym, einem Antikörper oder einem funktionellen Fragment oder Derivat davon oder einem auf einem Protein-A/Gold-, Protein-G/Gold- oder Avidin/-Streptavidin/Biotin-System beruht.

13. Medizinisches oder diagnostisches Gerät, das eine Oberfläche nach einem der vorstehenden Ansprüche aufweist.

14. Kit zur Verwendung mit einem Gerät nach Anspruch 13, der eine Oberfläche nach einem der vorstehenden Ansprüche und in Anspruch 11 definierte Nachweisreagenzien mit den in Anspruch 12 definierten Markierungen enthält.
